# EUROPEAN PATENT APPLICATION

(11) **EP 4 269 567 A1**
(43) Date of publication of application: **01.11.2023**
(21) Application number: 21911466.7
(22) Date of filing: 21.12.2021
(51) Int. Cl.: C12N 5/0775, A61K 35/28

(54) **STEM CELL PRIMING COMPOSITION AND PRIMED STEM CELL**

(30) Priority: 23.12.2020 KR 20200181940
(71) Applicant: SCM Lifescience Co., Ltd., Incheon 21999 (KR)
(72) Inventor: SONG, Sun Uk, Incheon 22003 (KR); KIM, Si Na, Incheon 21997 (KR)
(74) Representative: Boult Wade Tennant LLP
(86) International application number: PCT/KR2021/019485
(87) International publication number: WO 2022/139399

(57) **Abstract**

The present invention relates to a composition, comprising: TNF-α, IFN-y, and IFN-α; or TNF-α, IFN-y, IFN-α, and a vitamin, for enhancing immunomodulatory and inflammation-modulating ability of stem cells, and a method for preparing stem cells having improved immunomodulatory and inflammation-modulating ability by using same. Treatment of stem cells with: TNF-α, IFN-y, and IFN-α; or TNF-α, IFN-y, IFN-α, and a vitamin according to the present invention leads to upregulation of the expression of factors associated with the immunoregulatory activity and anti-inflammatory effects of stem cells and thus enables the preparation of functionally reinforced stem cells. The functionally reinforced stem cells thus prepared can find a wide spectrum of applications in various immune, inflammatory disease therapy fields.

## Description

### [Technical Field]

The present invention relates to a composition, comprising: TNF-α, IFN-y, and IFN-α; or TNF-α, IFN-y, IFN-α, and a vitamin, for enhancing immunomodulatory and inflammation-modulating ability of stem cells, and a method for preparing stem cells having improved immunomodulatory and inflammation-modulating ability by using same.

### [Background Art]

Stem cells are undifferentiated cells, which can divide for a long time through self-renewal, and refer to cells capable of differentiating into various types of cells under a specific environment. The stem cells may be divided into embryonic stem cells and adult stem cells depending on an originating tissue, but treatment experiments using the embryonic stem cells have difficulties due to ethical aspects and tumorigenicity, whereas the adult stem cells have an advantage of being easily obtained from various tissues, and thus, studies are being actively conducted to apply the stem cells to the treatment of various diseases.

To date, many compound immunosuppressive agents or anti-inflammatory agents have been developed, and the clinically most commonly used immunosuppressive agents include cyclosporine (Neoral, Cipol A), azathioprine (Imuran), prednisolone (a kind of steroid), and the like. The immunosuppressive agent inhibits several processes, such as phagocytosis of antigens by macrophages, antigen recognition by lymphocytes, cell division, division of T cells and B cells, and antibody production, during a process from antigen stimulation to antibody production, thereby causing immunosuppression. Most of these drugs have antitumor activity at the same time, but the reason is that these drugs inhibit cell division through DNA disorders, inhibition of DNA synthesis, and the like. However, representative side effects according thereto include hypertension and nephrotoxicity (decreased kidney function), and since the incidence of these side effects is high, there are problems such as a need to observe the progress sufficiently when used. Other side effects rarely include trembles, seizures, hepatitis, bile retention, increased uric acid in the blood, decreased muscle strength, hypertrichosis, gingival hypertrophy, and the like. Among the commonly used inhibitors, azathioprine inhibits bone marrow functions such as decreased white blood cell count, anemia, and decreased platelets, and may have complications such as pancreatitis, hepatitis, and bile retention, and rarely hair loss and fever. Prednisolone, one of the steroid drugs, started to be first used among immunosuppressive agents, but is a drug to be careful about not only enhancing arteriosclerosis, but also causing hypertension, gastric ulcer, diabetes, growth inhibition, osteoporosis, cataract, glaucoma, and the like. Therefore, the need for safe immunosuppressive agents or anti-inflammatory agents has emerged.

In order to solve these problems, therapies using stem cells have recently been attempted for the treatment of inflammatory and immune diseases. In particular, mesenchymal stem cells (MSCs) are known to suppress inflammation, induce the generation of regulatory T cells (Treg), or induce the death of immune cells involved in apoptosis, and thus, the development of various therapeutic agents using the MSCs is being actively conducted.

However, in various clinical trials using stem cells, there have been reported problems that persistence of effects or *in vivo* survival rates as a result of long-term follow-up was extremely low, and thus, various attempts are being made to improve stem cell therapeutics. For example, in order to increase the therapeutic effect, a method of pre-processing various chemicals, peptides, and the like capable of inserting specific genes into stem cells or boosting stem cell functions, and methods of adding stimulation conditions such as hypoxia, temperature, and light during culture are being tried, or in order to increase the survival rate of stem cells, methods of administering the stem cells together with a support are being studied. Among various studies for reinforcing the stem cell functions, a function improvement method using genetic manipulation may be effective, but problems such as the safety of the introduced gene are pointed out.

Therefore, various studies have been attempted to reinforce the intrinsic functions of stem cells through treatment of various priming factors during culture rather than reinforcing the functions by introducing the genes into the stem cells themselves.

### [Disclosure]

### [Technical Problem]

Accordingly, the present inventors confirmed that TNF-α, IFN-γ and IFN-α; or TNF-α, IFN-y, IFN-α and a vitamin were treated to effectively reinforce the functions of stem cells while studying various priming methods for enhancing inflammation-modulating ability and immunomodulatory of the stem cells, and then completed the present invention.

Accordingly, an object of the present invention is to provide a composition, including: TNF-α, IFN-y, and IFN-α; or TNF-α, IFN-y, IFN-α, and a vitamin, for enhancing immunomodulatory and inflammation-modulating ability of stem cells, a method for preparing stem cells having improved immunomodulatory and inflammation-modulating ability by using same, and stem cells having improved immunomodulatory and inflammation-modulating ability prepared by the method.

### [Technical Solution]

In order to achieve the object, the present invention provides a composition for enhancing immunomodulatory and inflammation-modulating ability of stem cells, comprising TNF-α, IFN-γ and IFN-α.

Further, the present invention provides a composition for enhancing immunomodulatory and inflammation-modulating ability of stem cells, comprising TNF-α, IFN-y, IFN-α, and at least one vitamin selected from the group consisting of vitamin B2, vitamin B3, vitamin B5 and vitamin B6.

Further, the present invention provides a use for enhancing immunomodulatory and inflammation-modulating ability of stem cells of TNF-α, IFN-y, IFN-α, and at least one vitamin selected from the group consisting of vitamin B2, vitamin B3, vitamin B5 and vitamin B6.

Further, the present invention provides an adjuvant composition for enhancing immunomodulatory and inflammation-modulating ability of stem cells, comprising at least one vitamin selected from the group consisting of vitamin B2, vitamin B3, vitamin B5 and vitamin B6.

Further, the present invention provides an adjuvant use for enhancing immunomodulatory and inflammation-modulating ability of stem cells, comprising at least one vitamin selected from the group consisting of vitamin B2, vitamin B3, vitamin B5 and vitamin B6.

Further, the present invention provides a method for enhancing immunomodulatory and inflammation-modulating ability of stem cells, including 1) treating and culturing stem cells with TNF-α, IFN-γ and IFN-α.

Further, the present invention provides a method for enhancing immunomodulatory and inflammation-modulating ability of stem cells, comprising treating at least one vitamin selected from the group consisting of vitamin B2, vitamin B3, vitamin B5 and vitamin B6 together in step 1).

Further, the present invention provides a method for preparing stem cells having improved immunomodulatory and inflammation-modulating ability, comprising 1) treating and culturing stem cells with TNF-α, IFN-γ and IFN-α.

Further, the present invention provides a method for enhancing immunomodulatory and inflammation-modulating ability of stem cells, comprising treating at least one vitamin selected from the group consisting of vitamin B2, vitamin B3, vitamin B5 and vitamin B6 together in step 1).

Further, the present invention provides a method for preparing stem cells having improved immunomodulatory and inflammation-modulating ability, comprising 1) treating and culturing stem cells with TNF-α, IFN-γ and IFN-α.

Further, the present invention provides a method for preparing stem cells having improved immunomodulatory and inflammation-modulating ability, comprising treating at least one vitamin selected from the group consisting of vitamin B2, vitamin B3, vitamin B5 and vitamin B6 together in step 1).

Further, the present invention provides stem cells having improved immunomodulatory and inflammation-modulating ability prepared by the preparing method.

### [Advantageous Effects]

According to the present invention, treatment of stem cells with: TNF-α, IFN-y, and IFN-α; or TNF-α, IFN-y, IFN-α, and a vitamin leads to upregulation of the expression of factors associated with the immunoregulatory activity and anti-inflammatory effects of stem cells and thus enables the preparation of functionally reinforced stem cells. The functionally reinforced stem cells thus prepared can find a wide spectrum of applications in various immune, and inflammatory disease therapy fields.

### [Description of Drawings]

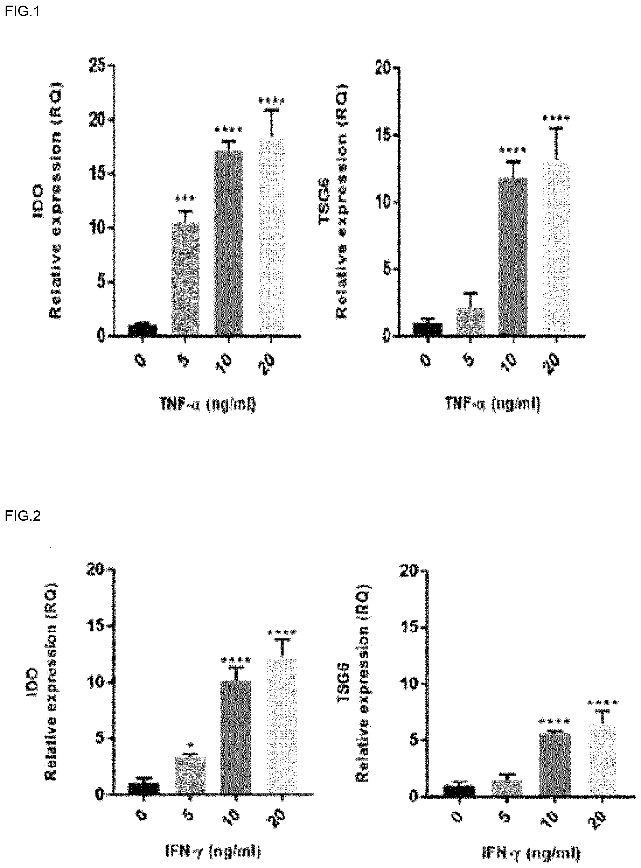

FIG. 1 is a diagram illustrating results of confirming increased expression of IDO and TSG6 according to a treatment concentration of TNF-α (***P < 0.001, ****P < 0.0001, compared to the control (0 ng/ml)).
FIG. 2 is a diagram illustrating results of confirming increased expression of IDO and TSG6 according to a treatment concentration of IFN-y (*P < 0.05, ^{∗∗∗∗}P < 0.0001, compared to the control (0 ng/ml)).

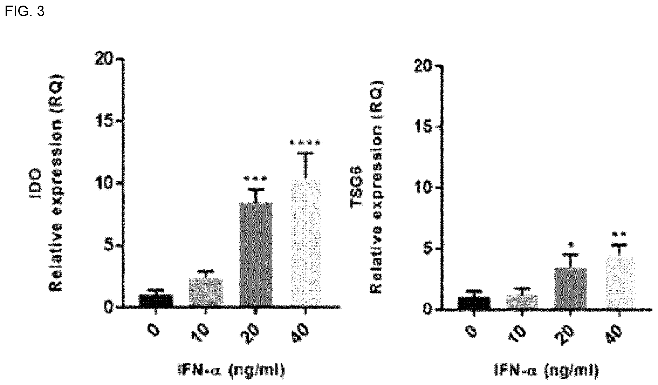

FIG. 3 is a diagram illustrating results of confirming increased expression of IDO and TSG6 according to a treatment concentration of IFN-α (*P < 0.05, **P < 0.01, ^{∗∗∗}P < 0.001, ^{∗∗∗∗}P < 0.0001, compared to the control (0 ng/ml)).

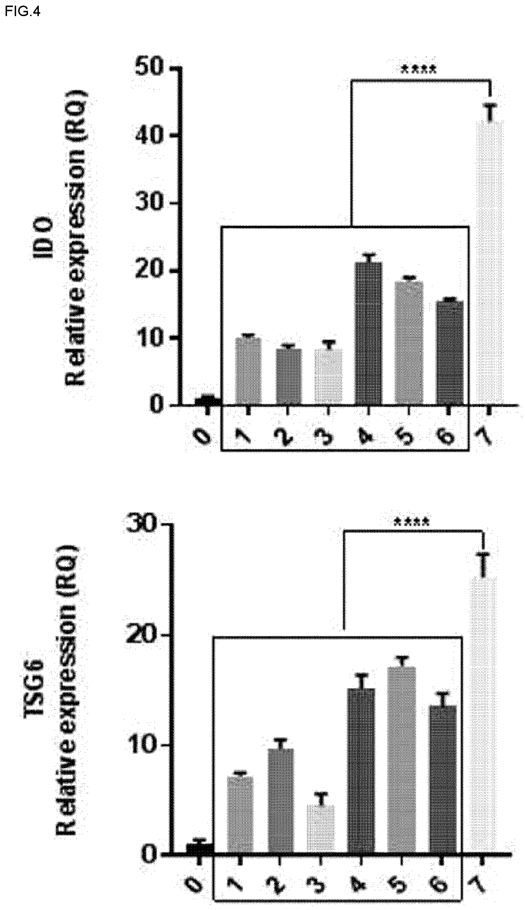

FIG. 4 is a diagram illustrating results of confirming increased expression of IDO and TSG6 according to compositions shown in Table 1 (****P < 0.0001) (0: untreated control; 1: TNF-α (10 ng/ml); 2: IFN-y (10 ng/ml); 3: IFN-α (20 ng/ml); 4: TNF-α (10 ng/ml) + IFN-y (10 ng/ml); 5: TNF-α (10 ng/ml) + IFN-α (20 ng/ml); 6: IFN-γ (10 ng/ml) + IFN-α (20 ng/ml); 7: TNF-α (10 ng/ml) + IFN-γ (10 ng/ml) + IFN-α (20 ng/ml)).

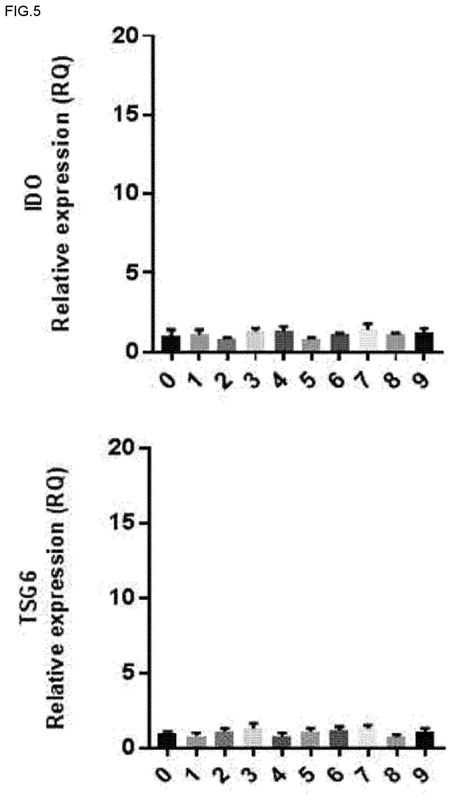

FIG. 5 is a diagram illustrating results of confirming increased expression of IDO and TSG6 according to single treatment of vitamins shown in Table 2 (0: untreated control; 1: Vitamin A (10 µg/ml); 2: Vitamin B1 (50 µg/ml); 3: Vitamin B2 (5 µg/ml); 4: Vitamin B3 (50 µg/ml); 5: Vitamin B5 (50 µg/ml); 6: Vitamin B6 (50 µg/ml); 7: Vitamin B12 (50 µg/ml); 8: Vitamin D2 (10 µg/ml); 9: Vitamin D3 (10 µg/ml)).

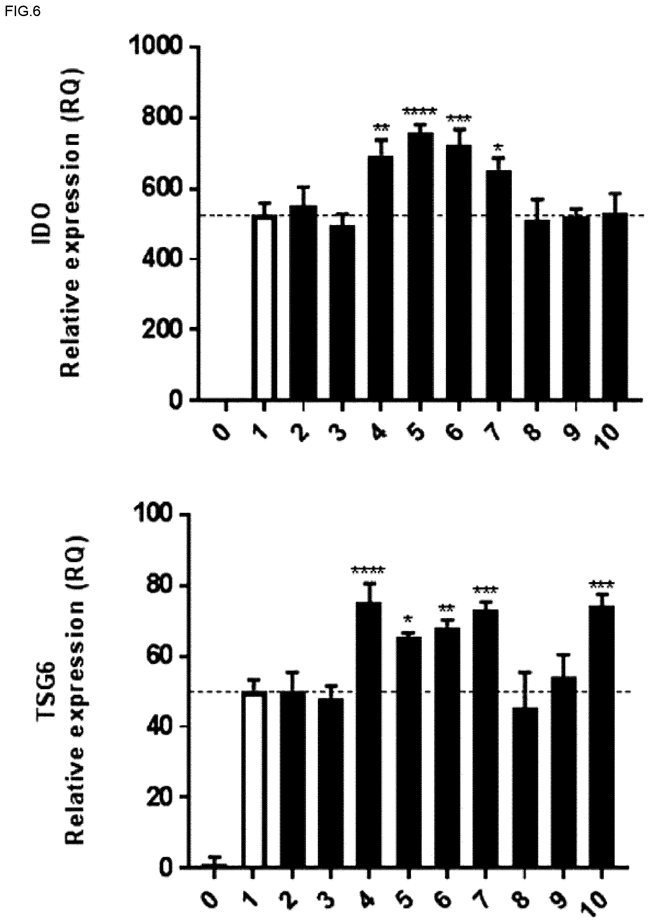

FIG. 6 is a diagram illustrating results of confirming increased expression of IDO and TSG6 according to treatment with compositions shown in Table 3 in which vitamins are added to TNF-α, IFN-y, and IFN-α, respectively. (*P < 0.05, **P < 0.01, ^{∗∗∗}P < 0.001, ^{∗∗∗∗}P < 0.0001, compared to group 1) (0: untreated control; 1: TNF-α + IFN-γ + IFN-α; 2: addition of vitamin A to Experimental Group 1; 3: addition of vitamin B1 to Experimental Group 1; 4: addition of vitamin B2 to Experimental Group 1; 5: addition of vitamin B3 to Experimental Group 1; 6: addition of vitamin B5 to Experimental Group 1; 7: addition of vitamin B6 to Experimental Group 1; 8: addition of vitamin B12 to Experimental Group 1; 9: addition of vitamin D2 to Experimental Group 1; 10: addition of vitamin D3 to Experimental Group 1).

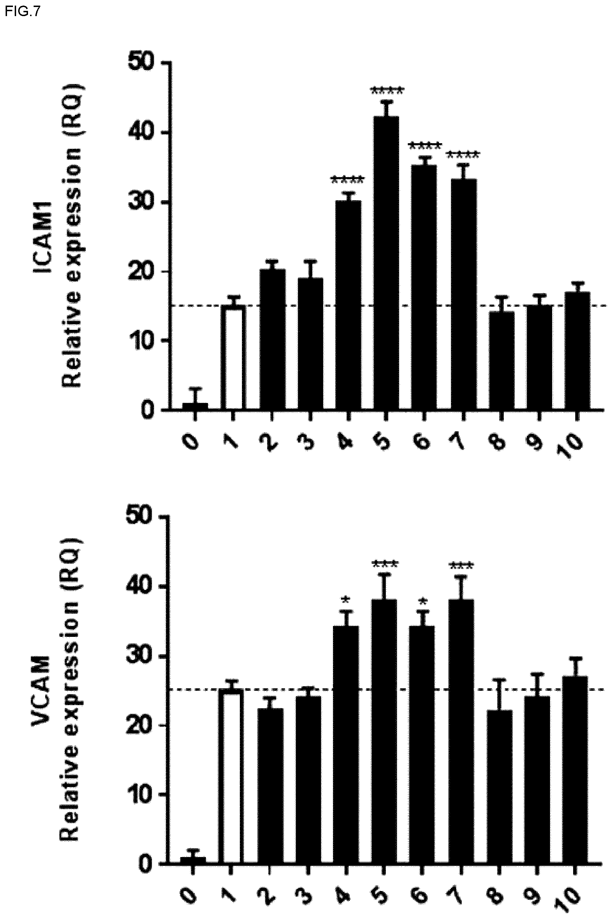

FIG. 7 is a diagram illustrating results of confirming increased expression of ICAM1 and VCAM according to treatment with compositions shown in Table 3 in which vitamins are added to TNF-α, IFN-y, and IFN-α, respectively. (*P < 0.05, **P < 0.01, ^{∗∗∗}P < 0.001, ^{∗∗∗∗}P < 0.0001, compared to group 1) (0: untreated control; 1: TNF-α + IFN-γ + IFN-α; 2: addition of vitamin A to Experimental Group 1; 3: addition of vitamin B1 to Experimental Group 1; 4: addition of vitamin B2 to Experimental Group 1; 5: addition of vitamin B3 to Experimental Group 1; 6: addition of vitamin B5 to Experimental Group 1; 7: addition of vitamin B6 to Experimental Group 1; 8: addition of vitamin B12 to Experimental Group 1; 9: addition of vitamin D2 to Experimental Group 1; 10: addition of vitamin D3 to Experimental Group 1)

### [Modes of the Invention]

Hereinafter, the present invention will be described in detail.

According to an aspect of the present invention, there is provided a composition including TNF-α, IFN-γ and IFN-α; or TNF-α, IFN-y, IFN-α and a vitamin, for enhancing immunomodulatory and inflammation-modulating ability of stem cells.

The composition of the present invention is a priming composition capable of priming the functions of stem cells, and when the stem cells are treated with TNF-α, IFN-γ and IFN-α; or TNF-α, IFN-y, IFN-α and a vitamin, functionally reinforced stem cells may be induced by reinforcing immunomodulatory and inflammation-modulating ability of stem cells.

The combination of TNF-α, IFN-γ and IFN-α; or TNF-α, IFN-y, IFN-α, and the vitamin exhibits a synergistic effect compared to treating stem cells with a single ingredient thereof, and may effectively induce a desired reinforced function of stem cells even at a low concentration.

Specifically, in order to induce the reinforced functions of the stem cells, the stem cells may be treated with TNF-α, IFN-y, and IFN-α and then cultured, and TNF-α, IFN-y, and IFN-α may be included in the composition in a ratio of 1 : 1 : 0.1 to 3 (w/v), preferably 1 : 1 : 1 to 3 (w/v), and more preferably 1 : 1 : 1 to 2.5 (w/v). In one embodiment of the present invention, it was confirmed that the stem cells were treated with TNF-α, IFN-y, and IFN-α at a concentration combination of 10 ng/ml, 10 ng/ml, and 20 ng/ml, and cultured to reinforce the functions of the stem cells.

In addition, the composition of the present invention may further include a vitamin in addition to the combination in order to more significantly induce the reinforced functions of the stem cells, and specifically, further include at least one vitamin selected from the group consisting of vitamin B2, vitamin B3, vitamin B5 and vitamin B6. When the stem cells are primed by treatment with the composition further containing the vitamin, the reinforced functions of the stem cells induced by treatment with TNF-α, IFN-γ and IFN-α may be more remarkably enhanced. The TNF-α, IFN-y, IFN-α, and the vitamin may be included in a ratio of 1 : 1 : 0.1 to 3 : 300 to 6,000 (w/v). More specifically, when vitamin B2 is added to TNF-α, IFN-y, and IFN-α, the TNF-α, IFN-y, IFN-α and the vitamin may be included in a ratio of 1 : 1 : 0.1 to 3 : 300 to 600 (w/v), more preferably 1 : 1 : 0.1 to 3 : 400 to 550 (w/v), and much more preferably 1 : 1 : 2 : 500 (w/v). In addition, when vitamin B3, vitamin B5, or vitamin B6 is added to TNF-α, IFN-y, and IFN-α, the TNF-α, IFN-y, IFN-α and the vitamin may be included in a ratio of 1 : 1 : 0.1 to 3 : 4,000 to 6,000 (w/v), more preferably 1 : 1 : 0.1 to 3 : 4,000 to 5,500 (w/v), and much more preferably 1 : 1 : 2 : 5,000 (w/v).

In one embodiment of the present invention, 10 ng/ml of TNF-α, 10 ng/ml of IFN-y, and 20 ng/ml of IFN-α were selected, and 5 µg/ml of vitamin B2, 50 µg/ml of vitamin B3, 50 µg/ml of vitamin B5 and 50 µg/ml of vitamin B6 were selected as concentrations for combined treatment, and treated to stem cells to confirm the reinforcement of immunomodulatory and inflammation-modulating ability. As a result, in Experimental Groups added with vitamins, compared to treatment of TNF-α, IFN-y, and IFN-α, it was confirmed that not only the expression of IDO and TSG6 was increased, but also the expression of intercellular adhesion molecule 1 (ICAM1) and vascular cell adhesion molecule (VCAM) as adhesion factors expressed on the surface of stem cells was significantly increased.

In the present invention, the stem cells functionally reinforced by treatment with TNF-α, IFN-y, and IFN-α; or TNF-α, IFN-y, IFN-α and the vitamin refer to cells that have ability to differentiate into two or more cells while having self-replication ability, and may be classified into totipotent stem cells, pluripotent stem cells, and multipotent stem cells. The stem cells may be appropriately selected without limitation depending on the purpose, and may be derived from adult cells, such as all known tissues, cells, and the like derived from mammals including humans, preferably humans, and for example, mesenchymal stem cells derived from fat, bone marrow, placenta (or placental tissue cells), or umbilical cord blood.

When the stem cells are treated with TNF-α, IFN-γ and IFN-α; or TNF-α, IFN-y, IFN-α and the vitamin and then cultured, the functions of the stem cells may be reinforced.

In the present invention, "the reinforced function of the stem cells" has the same meaning as "priming of stem cells", and means increasing usefulness by further boosting the inherent properties and effects of stem cells. More specifically, the reinforced function preferably means boosting the inflammation-modulating ability and immunomodulatory inherently present in stem cells, and at this time, TNF-α, IFN-γ and IFN-α; or TNF-α, IFN-y, IFN-α and the vitamin may be used as a priming agent to boost stem cells.

More specifically, the reinforced function refers to reinforcement of inflammation-modulating ability and immunomodulatory and may mean enhancing immunomodulatory and inflammation-modulating ability of stem cells of at least one selected from the group consisting of increased expression of TNFα-stimulated gene-6 (TSG6), increased expression of indoleamine 2,3-dioxygenase (IDO), increased expression of intercellular adhesion molecule 1 (ICAM1), and increased expression of vascular cell adhesion molecule (VCAM).

Further, the present invention provides an adjuvant composition including at least one vitamin selected from the group consisting of vitamin B2, vitamin B3, vitamin B5 and vitamin B6, for enhancing immunomodulatory and inflammation-modulating ability of stem cells.

The vitamin B2, vitamin B3, vitamin B5 and vitamin B6 of the present invention cannot promote the immunomodulatory or inflammation-modulating ability of stem cells when treated to stem cells alone, but may significantly increase the immunomodulatory or inflammation-modulating ability of the stem cells when treated with a priming agent capable of reinforcing the functions of stem cells. Therefore, at least one vitamin selected from the group consisting of vitamin B2, vitamin B3, vitamin B5 and vitamin B6 may be treated with a stem cell priming agent known in the art, preferably a priming agent capable of enhancing the immunomodulatory or inflammation-modulating ability of stem cells, for the purpose of combined treatment.

In one embodiment of the present invention, in addition to TNF-α, IFN-y, and IFN-α, the vitamins are additionally treated to confirm the synergistic effect, and accordingly, the enhancement of the immunomodulatory and the inflammation-modulating ability of stem cells may be immunomodulatory and inflammation-modulating ability of stem cells induced by treatment of TNF-α, IFN-y, and IFN-α.

In the present invention, when at least one vitamin selected from the group consisting of vitamin B2, vitamin B3, vitamin B5 and vitamin B6 is used as an adjuvant for enhancing the immunomodulatory and inflammation-modulating ability of stem cells, these vitamins may be treated to the stem cells at a concentration of 0.1 to 300 µg/ml, 1 to 300 µg/ml, 3 to 100 µg/ml, preferably 3 to 60 µg/ml.

In the present invention, when at least one vitamin selected from the group consisting of vitamin B2, vitamin B3, vitamin B5, and vitamin B6 is treated with TNF-α, IFN-y, and IFN-α in combination, the vitamin may be treated in combination in the following ratio. The TNF-α, IFN-y, IFN-α and the vitamin may be treated in a ratio of 1 : 1 : 0.1 to 3 : 300 to 6,000 (w/v). More specifically, when vitamin B2 is added to TNF-α, IFN-y, or IFN-α, the TNF-α, IFN-y, IFN-α and the vitamin may be treated in a ratio of 1 : 1 : 0.1 to 3 : 300 to 600 (w/v), more preferably 1 : 1 : 0.1 to 3 : 400 to 550 (w/v), and much more preferably 1 : 1 : 2 : 500 (w/v). In addition, when vitamin B3, vitamin B5, or vitamin B6 is added to TNF-α, IFN-y, and IFN-α, the TNF-α, IFN-y, IFN-α and the vitamin may be treated in a ratio of 1 : 1 : 0.1 to 3 : 4,000 to 6,000 (w/v), more preferably 1 : 1 : 0.1 to 3 : 4,000 to 5,500 (w/v), and much more preferably 1 : 1 : 2 : 5,000 (w/v).

Further, the present invention provides a method for enhancing immunomodulatory and inflammation-modulating ability of stem cells, including 1) treating and culturing stem cells with TNF-α, IFN-γ and IFN-α.

Further, the present invention provides a method for preparing stem cells having improved immunomodulatory and inflammation-modulating ability, including 1) treating and culturing stem cells with TNF-α, IFN-γ and IFN-α.

In the present invention, in order to further promote the immunomodulatory and inflammation-modulating ability, in addition to TNF-α, IFN-y, and IFN-α, at least one vitamin selected from the group consisting of vitamin B2, vitamin B3, vitamin B5, and vitamin B6 may be treated together, and these vitamins may be treated simultaneously or sequentially.

The method may be performed *in vitro* or *ex vivo,* and the culture may be performed in a medium containing ingredients necessary for culturing stem cells well-known in the art or a medium additionally containing ingredients capable of enhancing the proliferation of stem cells. The stem cells of the present invention can be grown in a general medium, and the medium contains nutrients required by cells as an object to be cultured, that is, an embryoid body to culture the stem cells of the present invention, and may be additionally added and mixed with materials for special purposes. The medium may also be referred to as a culture medium or a culture solution, and is a concept that includes all natural media, synthetic media, or selective media. For example, in the present invention, a Dulbecco's modified Eagle medium (DMEM) or alpha-MEM may be used, but is not limited thereto. In addition, culture conditions such as temperature and culture time may follow conventional culture conditions of mesenchymal stem cells.

In the method for enhancing the immunomodulatory and inflammation-modulating ability of stem cells and the method for preparing the stem cells having improved immunomodulatory and inflammation-modulating ability, the enhancement of the immunomodulatory and inflammation-modulating ability of stem cells may be at least one selected from the group consisting of increased expression of TNFa-stimulated gene-6 (TSG6), increased expression of indoleamine 2,3-dioxygenase (IDO), increased expression of intercellular adhesion molecule 1 (ICAM1), and increased expression of vascular cell adhesion molecule (VCAM) of the stem cells.

In the method of the present invention, the contents overlapping with the composition for enhancing the immunomodulatory and inflammation-modulating ability are omitted to avoid complexity in the description.

Further, the present invention provides stem cells having improved immunomodulatory and inflammation-modulating ability prepared by the preparing method.

The stem cells according to the present invention are functionally reinforced stem cells, and have the same meaning as primed stem cells. The stem cells according to the present invention are stem cells with further boosted inherent properties and effects of the stem cells, compared to stem cells that are not treated with the priming material. Preferably, when TNF-α, IFN-γ and IFN-α; or TNF-α, IFN-y, IFN-α and a vitamin are treated in the culture process, the stem cells may be stem cells with boosted inflammation-modulating ability and immunomodulatory inherently possessed by stem cells.

More specifically, in the stem cells of the present invention, the expression of at least one selected from the group consisting of TSG6, IDO, ICAM1 and VCAM is enhanced.

When the stem cells of the present invention are used as a pharmaceutical composition, the pharmaceutical composition may further include suitable carriers, excipients and diluents commonly used in the preparation of the pharmaceutical composition. In addition, additives for solid or liquid formulations may be used in the preparation of the pharmaceutical composition. The additives for formulations may be either organic or inorganic.

Examples of the excipient may include lactose, sucrose, white sugar, glucose, corn starch, starch, talc, sorbitol, crystalline cellulose, dextrin, kaolin, calcium carbonate, silicon dioxide, and the like. Examples of the binder may include polyvinyl alcohol, polyvinyl ether, ethyl cellulose, methyl cellulose, Arabic gum, tragacanth, gelatin, shellac, hydroxypropyl cellulose, hydroxypropyl methyl cellulose, calcium citrate, dextrin, pectin, and the like. Examples of the lubricant may include magnesium stearate, talc, polyethylene glycol, silica, hydrogenated vegetable oil, and the like. As a coloring agent, any coloring agent may be used as long as the coloring agent is permitted to be added to ordinary pharmaceuticals. These tablets and granules may be sugar-coated, gelatin-coated, or appropriately coated as needed. In addition, preservatives, antioxidants, etc. may be added as needed.

The pharmaceutical composition of the present invention may be prepared in any formulation commonly prepared in the art, and the form of the formulation is not particularly limited, but may be preferably external preparations. The external preparations of the present invention may include conventional external preparations such as sheets, liquid coating agents, sprays, lotions, creams, puff paste, powders, penetration pads, sprays, gels, pastas, liniments, ointments, aerosols, powders, suspensions, and transdermal absorbents. These formulations are described in prescriptions generally known to all pharmaceutical chemistries.

The pharmaceutically effective amount of the present invention may vary depending on a patient's wound type, an application site, a treatment frequency, treatment time, a dosage form, a patient's condition, a type of adjuvant, and the like. The used amount is not particularly limited, but may be 0.00001 to 10000 µg when the daily effective amount of the pharmaceutical composition of the present invention is applied to a patient. The daily dose may be administered once a day, divided into 2 to 3 times a day at appropriate intervals, or may be administered intermittently at intervals of several days.

However, since the used amount of the pharmaceutical composition of the present invention is determined according to many related factors such as a route of administration, age, sex, weight of a patient, patient's severity, a type of wound, an application site, treatment frequency, treatment time, a dosage form, a patient's condition, a type of adjuvant, and the like, the effective amount should not be understood as limiting the scope of the present invention in any respect.

Hereinafter, the present invention will be described in more detail through Examples. These Examples are just illustrative of the present invention, and it will be apparent to those skilled in the art that it is not interpreted that the scope of the present invention is limited to these Examples.

### Example 1. Culture of stem cells and selection of candidate substances

In a 37°C and 5% CO₂ incubator, mesenchymal stem cells in a storage state (LN2 tank storage) were thawed and cultured, and at this time, the cells were cultured in a medium (DMEM, alpha-MEM) containing 10% FBS or 4% hPL until proliferated to a cell confluence of about 80%. The cultured mesenchymal stem cells were seeded in a 100 mm dish, and treated with candidate substances for reinforcing the functions of the mesenchymal stem cells for 24 hours, and then the concentrations of primary candidate substances for reinforcing the functions were set.

TNF-α and IFN-γIFN-α were selected as the primary candidate substances, and in order to confirm the lowest effective concentration capable of increasing the expression of TNFa-stimulated gene-6 (TSG6) and indoleamine 2,3-dioxygenase (IDO) to confirm the reinforced functions, the stem cells were treated with 5, 10, and 20 ng/ml of TNF-α, 5, 10, and 20 ng/ml of IFN-y, and 10, 20, and 40 ng/ml of IFN-α, respectively, and changes in expression of TSG6 and IDO were confirmed.

The IDO was known as an immunomodulatory factor that inhibited the proliferation of immune cells such as T cells by converting tryptophan, which was essential for T cell proliferation, into kynurenine, and the TSG6 was known as an anti-inflammatory regulator secreted by mesenchymal stem cells. After culturing the stem cells, total RNA was isolated using TRIzol (Invitrogen), and then cDNA was synthesized from total RNA using PrimeScript^{™}RT reagent Kit with gDNA Eraser (TaKaRa), and qRT-PCR was performed. The results of TSG6 and IDO according to the concentration of each candidate substance were illustrated in FIGS. 1, 2 and 3.

As illustrated in FIGS. 1 to 3, 10 ng/ml of TNF-α, 10 ng/ml of IFN-y, and 20 ng/ml of IFN-α were confirmed as the lowest effective concentrations capable of inducing significantly increased expression of both TSG6 and IDO, and thereafter, the experiment was performed based on the corresponding concentrations.

### Example 2. Selection of combination for reinforcing functions of stem cells

Through Example 1, candidate substances and concentration combinations thereof selected to search for more functionally reinforced compositions based on the candidate substances and the lowest effective concentrations confirmed to induce the reinforced functions of stem cells were set as shown in Table 1. Mesenchymal stem cells were treated with the candidate combinations described in Table 1 below for 24 hours, and the stem cells were cultured as in Example 1, and then total RNA was isolated using TRIzol (Invitrogen). Then, qRT-PCR was performed by synthesizing cDNA from total RNA using the PrimeScript^{™} RT reagent Kit with gDNA Eraser (TaKaRa).

**[Table 1]**

| Experimental group | |
|---|---|
| 0 | Control (untreated group) |
| 1 | TNF-α (10 ng/ml) |
| 2 | IFN-γ (10 ng/ml) |
| 3 | IFN-α (20 ng/ml) |
| 4 | TNF-α (10 ng/ml) + IFN-γ (10 ng/ml) |
| 5 | TNF-α (10 ng/ml) + IFN-α (20 ng/ml) |
| 6 | IFN-γ (10 ng/ml) + IFN-α (20 ng/ml) |
| 7 | TNF-α (10 ng/ml) + IFN-γ (10 ng/ml) + IFN-α (20 ng/ml) |

Through this, changes in the expression of IDO and TSG6, which were representative factors of immune and anti-inflammatory regulation, were confirmed, and the results were shown in FIG. 4.

As illustrated in FIG. 4, compared to an untreated control group 0, increased expression of IDO and TSG6 was confirmed in treatment groups of all single candidate substances, TNF-α, IFN-y, and IFN-α (Experimental groups 1 to 3), but in Experimental Groups 4 to 6 in which these substances were combined, a more significant increase in expression of IDO and TSG6 was confirmed compared to the single candidate substance-treated groups. In particular, Experimental Groups 4 and 5, in which TNF-α was treated with IFN-γ or IFN-α, excellent effects were exhibited compared to Experimental Group 6, in which only IFN-γ and IFN-α were combined, and surprisingly, in a treatment group in which all three candidate substances were combined, a function reinforcing effect which was twice or more increased than that of a combination of two substances was confirmed.

Therefore, through the results, when the stem cells were treated with a combination of TNF-α (10 ng/ml) + IFN-γ (10 ng/ml) + IFN-α (20 ng/ml), it was confirmed that very significantly reinforced immunity and anti-inflammatory functions of the stem cells could be achieved.

### Example 3. Confirmation of function reinforcing effect of stem cells by adding vitamins

### 3.1 Confirmation of function reinforcing effect of stem cells by adding vitamin alone

Vitamins were known to improve the proliferative ability of stem cells and maintain the sternness of stem cells when added during a stem cell culture process. Accordingly, various vitamins were treated in the stem cell culture process, and experiments were performed to confirm whether or not vitamins could achieve anti-inflammatory and immune function reinforcing effects of stem cells. Specifically, the stem cells were treated with various types of vitamins shown in Table 2 according to the method described in Example 1, and the results of confirming changes in expression of IDO and TSG6 according to treatment with each candidate substance were illustrated in FIG. 5.

**[Table 2]**

| Experimental group | |
|---|---|
| 0 | Control (untreated group) |
| 1 | vitamin A 10 µg/ml |
| 2 | vitamin B1 50 µg/ml |
| 3 | vitamin B2 5 µg/ml |
| 4 | vitamin B3 50 µg/ml |
| 5 | vitamin B5 50 µg/ml |
| 6 | vitamin B6 10 µg/ml |
| 7 | vitamin B12 50 µg/ml |
| 8 | vitamin D2 10 µg/ml |
| 9 | vitamin D3 10 µg/ml |

As illustrated in FIG. 5, the vitamin-treated groups did not show significant effects on the changes in expression of IDO and TSG6 involved in the anti-inflammatory and immunomodulatory functions of stem cells.

### 3.2 Confirmation of stem cell function reinforcing effect according to combination of vitamin and TNF-α + IFN-γ + IFN-α

As described above, when vitamins, which did not show an effect of reinforcing the anti-inflammatory and immunomodulatory functions of the stem cells as a single substance, were added to a combination of functionally reinforced substances identified in Example 2, in order to confirm whether or not to exhibit a synergistic effect, as shown in Table 3 below, each vitamin was added to TNF-α (10 ng/ml) + IFN-γ (10 ng/ml) + IFN-α (20 ng/ml), expression changes of IDO and TSG6 according to each combination treatment were confirmed, and the results were shown in FIG. 6.

**[Table 3]**

| Experimental group | |
|---|---|
| 0 | Control (untreated group) |
| 1 | TNF-α (10 ng/ml) + IFN-γ (10 ng/ml) + IFN-α (20 ng/ml) |
| 2 | TNF-α (10 ng/ml) + IFN-γ (10 ng/ml) + IFN-α (20 ng/ml) + vitamin A 10 ug/ml |
| 3 | TNF-α (10 ng/ml) + IFN-γ (10 ng/ml) + IFN-α (20 ng/ml) + vitamin B1 50 ug/ml |
| 4 | TNF-α (10 ng/ml) + IFN-γ (10 ng/ml) + IFN-α (20 ng/ml) + vitamin B2 5 ug/ml |
| 5 | TNF-α (10 ng/ml) + IFN-γ (10 ng/ml) + IFN-α (20 ng/ml) + vitamin B3 50 ug/ml |
| 6 | TNF-α (10 ng/ml) + IFN-γ (10 ng/ml) + IFN-α (20 ng/ml) + vitamin B5 50 ug/ml |
| 7 | TNF-α (10 ng/ml) + IFN-γ (10 ng/ml) + IFN-α (20 ng/ml) + vitamin B6 50 ug/ml |
| 8 | TNF-α (10 ng/ml) + IFN-γ (10 ng/ml) + IFN-α (20 ng/ml) + vitamin B12 50 ug/ml |
| 9 | TNF-α (10 ng/ml) + IFN-γ (10 ng/ml) + IFN-α (20 ng/ml) + vitamin D2 10 ug/ml |
| 10 | TNF-α (10 ng/ml) + IFN-γ (10 ng/ml) + IFN-α (20 ng/ml) + vitamin D3 10 ug/ml |

As shown in FIG. 6, the expression of IDO and TSG6 was not significantly increased in Experimental Groups 2, 3, 8, and 9 compared to Experimental Group 1 without vitamin treatment, and in Experimental Group 10, the expression of TSG6 was increased, but the increase in expression of IDO was not confirmed compared to the control group. On the other hand, in the case of vitamin B2 (Experimental Group 4), vitamin B3 (Experimental Group 5), vitamin B5 (Experimental Group 6), and vitamin B6 (Experimental Group 7), it was confirmed that the expression of both IDO and TSG6 was increased significantly. It was confirmed that in the case of TSG6, as compared to Experimental Group 1 treated with TNF-α + IFN-γ + IFN-α, in Experimental Groups 4, 5, 6, and 7, the expression was increased by 49%, 30%, 35%, and 45%, respectively, and in the case of IDO, as compared to Experimental Group 1, in Experimental Groups 4, 5, 6, and 7, the expression was increased by 25%, 37%, 31%, and 18%, respectively. As a result, in the case of treating stem cells with vitamins alone, the anti-inflammatory and immunomodulatory abilities of stem cells were not improved, but in the case of treating a combination of TNF-α + IFN-γ + IFN-α and vitamin B2, vitamin B3, vitamin B5 or vitamin B6, the effect of TNF-α + IFN-γ + IFN-α may be further enhanced.

### Example 4. Confirmation of expression changes of ICAM1 and VCAM

ICAM1 and VCAM may exhibit effects on various immune and inflammatory-related diseases by inhibiting proliferation and inducing death of T cells through T cell binding to reduce excessive immune and inflammatory responses. Accordingly, mesenchymal stem cells were treated for 24 hours with the same Experimental Group described in Table 3 of Example 3 above, and the stem cells were cultured as in Example 1, and then total RNA was isolated using TRIzol (Invitrogen). Then, qRT-PCR was performed by synthesizing cDNA from total RNA using the PrimeScript^{™} RT reagent Kit with gDNA Eraser (TaKaRa).

In order to confirm the enhancement of the effect of stem cells for treating immune diseases and inflammatory diseases, the expression of intercellular adhesion molecule 1 (ICAM1) and vascular cell adhesion molecule (VCAM), which were adhesion factors expressed on the surface of stem cells, was confirmed, and the results were illustrated in FIG. 7.

As illustrated in FIG. 7, it was confirmed that in the case of ICAM1, compared to Experimental Group 1 treated with TNF-α + IFN-γ + IFN-α, in Experimental Groups 4, 5, 6, and 7, the expression was increased by 45%, 100%, 35%, and 45%, respectively, and in the case of VACM, compared to Experimental Group 1, in Experimental Groups 4, 5, 6, and 7, the expression was increased by 52%, 69%, 52%, and 69%, respectively.

Therefore, when the stem cells were treated with a combination of TNF-α + IFN-γIPN-α and vitamin B2, vitamin B3, vitamin B5 or vitamin B6, the expression of ICAM1 and VCAM was significantly increased, and as a result, the immunomodulatory activity and the anti-inflammatory effect of the stem cells may be promoted.

When combining the experimental results, it can be seen that when the stem cells are treated with TNF-α, IFN-γ and IFN-α; or TNF-α, IFN-y, IFN-α and a vitamin, the immune and inflammation-modulating abilities of mesenchymal stem cells may be further improved. Accordingly, the combination of TNF-α, IFN-γ and IFN-α; or TNF-α, IFN-y, IFN-α and the vitamin may be used as a priming factor for reinforcing the inflammation-modulating ability and the immunomodulatory of mesenchymal stem cells.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A composition for enhancing immunomodulatory and inflammation-modulating ability of stem cells, comprising TNF-α, IFN-γ and IFN-α.

2. The composition of claim 1, wherein the TNF-α, IFN-γ and IFN-α are included in a ratio of 1 : 1 : 0.1 to 3 (w/v).

3. The composition of claim 1, further comprising: at least one vitamin selected from the group consisting of vitamin B2, vitamin B3, vitamin B5 and vitamin B6.

4. The composition of claim 3, wherein the TNF-α, IFN-y, IFN-α and the vitamin are included in a ratio of 1 : 1 : 0.1 to 3 : 300 to 6,000 (w/v).

5. The composition of any one of claims 1 to 4, wherein the stem cells are mesenchymal stem cells derived from fat, bone marrow, placenta or umbilical cord blood.

6. The composition of any one of claims 1 to 4, wherein the composition induces enhancement of immunomodulatory and inflammation-modulating ability of stem cells of at least one selected from the group consisting of TNFα-stimulated gene-6 (TSG6) expression increase, indoleamine 2,3-dioxygenase (IDO) expression increase, intercellular adhesion molecule 1 (ICAM1) expression increase, and vascular cell adhesion molecule (VCAM) expression increase of the stem cells.

7. An adjuvant composition for enhancing immunomodulatory and inflammation-modulating ability of stem cells, comprising at least one vitamin selected from the group consisting of vitamin B2, vitamin B3, vitamin B5 and vitamin B6.

8. The adjuvant composition of claim 7, wherein the enhancement of the immunomodulatory and inflammation-modulating ability of stem cells is induced by treatment of TNF-α, IFN-γ and IFN-α.

9. The adjuvant composition of claim 8, wherein the vitamin is added so that the ratio of TNF-α, IFN-y, IFN-α and the vitamin is 1 : 1 : 0.1 to 3 : 300 to 6,000 (w/v).

10. A method for enhancing immunomodulatory and inflammation-modulating ability of stem cells, comprising 1) treating and culturing stem cells with TNF-α, IFN-γ and IFN-α.

11. The method of claim 10, wherein in step 1), at least one vitamin selected from the group consisting of vitamin B2, vitamin B3, vitamin B5 and vitamin B6 is treated together.

12. The method of claim 10, wherein the enhancement of the immunomodulatory and inflammation-modulating ability of the stem cells is at least one selected from the group consisting of TNFα-stimulated gene-6 (TSG6) expression increase, indoleamine 2,3-dioxygenase (IDO) expression increase, intercellular adhesion molecule 1 (ICAM1) expression increase, and vascular cell adhesion molecule (VCAM) expression increase of the stem cells.

13. A method for preparing stem cells having improved immunomodulatory and inflammation-modulating ability, comprising 1) treating and culturing stem cells with TNF-α, IFN-γ and IFN-α.

14. The method of claim 13, wherein in step 1), at least one vitamin selected from the group consisting of vitamin B2, vitamin B3, vitamin B5 and vitamin B6 is treated together.

15. A stem cell having improved immunomodulatory and inflammation-modulating ability prepared by the method of claim 13 or 14.

16. The stem cell of claim 15, wherein in the stem cell, expression of at least one selected from the group consisting of TSG6, IDO, ICAM1 and VCAM is enhanced.
